Europäisches Patentamt

European Patent Office

Office européen des brevets

(19)

(11) Publication number: **0 268 468**
**A2**

(12) # EUROPEAN PATENT APPLICATION

(21) Application number: 87310180.2

(22) Date of filing: 18.11.87

(51) Int. Cl.⁴: **C 12 N 15/00**
C 12 P 21/00, A 61 K 39/395,
A 61 K 49/02, G 01 N 33/577

(30) Priority: 19.11.86 DK 5548/86

(43) Date of publication of application:
25.05.88 Bulletin 88/21

(84) Designated Contracting States:
AT BE CH DE ES FR GB GR IT LI LU NL SE

(71) Applicant: NOVO INDUSTRI A/S
Novo Allé
DK-2880 Bagsvaerd (DK)

(72) Inventor: Kjeldsen, Thomas Borglum
Gronnevej 87
DK-2830 Virum (DK)

Zeuthen, Jesper
Peder Godskesvej 8
DK-2830 Virum (DK)

(74) Representative: Raynor, John et al
W.H. Beck, Greener & Co 7 Stone Buildings Lincoln's Inn
London WC2A 3SZ (GB)

The applicant has filed a statement in accordance with Rule 28 (4) EPC (issue of a sample only to an expert). Accession number(s) of the deposit(s): ECACC 8611 1005, 1004, 1003, 1002.
The microorganisms have been deposited with ECACC under number(s) 8611/1005, 1004, 1003, 1002.

(54) **Antibodies.**

(57) Hybrid cell lines producing monoclonal antibodies reactive with antigenic determinants produced on the surface or in the cytoplasma of mammary carcinoma cells and secreted from the carcinoma cells.

The antibodies are useful in the diagnosis and therapy of especially mammary cancer.

EP 0 268 468 A2

**Description**

Antibodies

FIELD OF THE INVENTION

The present invention is related to monoclonal antibodies and more particularly to monoclonal antibodies reactive with antigens found on mammary tumor cell surfaces and in their cytoplasma.

BACKGROUND OF THE INVENTION

Antibodies have long been used in medical diagnosis, e.g., determining blood types, and in biological experimentation. With development of techniques of producing monoclonal antibodies which make it possible to obtain homogenous, highly specific antibodies, Kohler G. and Milstein, C.: (1975) Nature (London) 256 495 - 497, the utility of antibodies has been greatly increased. Unlike antibody fraction which were previously available and which were actually heterogeneous mixtures of a number of antibody molecules reactive with a variety of antigenic determinants, the molecules in a monoclonal antibody are all identical and are generally reactive with a single antigenic determinant or a group of closely related antigenic determinants. Monoclonal antibodies are therefore much more precise probes for detecting the presence of a particular substance than were previous heterogeneous antibody fractions. The precise selectivity of monoclonal antibodies makes them particularly useful for diagnostic purposes and even as therapeutic agents against selected biological material, such as tumor cells. Monoclonal antibodies have been used to detect and isolate biological substances which were previously unknown.

Generally, monoclonal antibodies are produced by immunizing an animal with a biological specimen or other foreign substance, obtaining antibody-producing cells from the animal, and fusing the antibody-producing cells with strains of neoplastic cells, e.g., tumor cells, to produce hybridomas which are isolated and cultured as monoclones. The monoclonal hybridomas may either be cultured in vitro or may be grown in vivo as tumors in a host animal. Because each antibody-producing cell produces a single unique antibody, the monoclonal cultures of hybridomas each produce a homogenous antibody fraction which may be obtained either from the culture medium of hybridoma cultures grown in vitro or from the ascitic fluid, or serum of a tumor-bearing host animal.

Not all of the hybridoma clones which result from fusing neoplastic cells with antibody-producing cells are specific for the desired foreign substance or antigen because many of the hybridomas will make antibodies which the animal's immune system has generated in reaction to other foreign substances. Even monoclonal antibodies against the subject antigen will differ from clone to clone because antibodies produced by different clones may react with different antigenic determinants of the same molecule. From each clone, therefore, it is necessary to obtain the resulting antibody or the antibody-containing medium, serum or ascitic fluid and test its reactivity with the subject biological material and to test its specificity by determining what other biological material, if any, it recognizes. While the necessity of characterizing the antibody of each clone adds to the complexity of producing monoclonal antibodies, the wide variety of homogenous antibodies which may be obtained gives investigators a number of very precise tools to map the structure and development of somatic cells.

A monoclonal antibody is produced which is specific for antigens found predominantly on human mammary tumor cells. Mice are inoculated with human mammary tumor cells, and spleen cells or lymph node cells are obtained from the inoculated mice and fused with mouse tumor cells. Monocultures of the fused cells are produced, and the antibodies obtained from the monoclones are tested for their ability to react with a variety of randomly obtained human mammary tumor tissues. In order to select a monoculture which produces an antibody with the desired characteristics, the reactivity of the antibody with other cells, including both normal human cells and other human tumor cells is investigated. A monoclonal antibody from one hybridoma clone is reactive with a mammary tumor cell surface antigen and is particularly useful for diagnosing mammary tumors. The antibody is potentially useful as an agent against mammary tumors.

Human mammary carcinoma currently represents the leading cause of cancer death in women in the United States with more than 150,000 new cases diagnosed each year. The histopathological classification of human mammary carcinomas is currently dependent on morphologic description alone. Approximately 80% are infiltrating ductal carcinomas, 10% are infiltrating lobular carcinomas, and the remaining 10% comprise a number of histologic types including intraductal carcinoma. Excepting inflammatory carcinoma, for the most part, morphology has not proven to be a good predictor of clinical prognosis or response to therapy. Indeed, extent of disease (tumor size and nodal positivity) have continued to determine clinical management. Recently, with the advent of estrogen and progesterone receptor determinations, correlations have begun to be made between clinical variables and biologic characteristics of malignant mammary cells.

SUMMARY OF THE INVENTION

In accordance with the invention, hybridomas were developed which produce monoclonal antibodies which are generally reactive with human mammary carcinoma cells, specifically reacting with cell surface antigens which are prevalent on mammary cell tumor surfaces and with a cytoplasmatic antigen, and where all the antigens recognized by the monoclonal antibodies of the invention are secreted from the tumor cells and thus present in the serum of a subject suffering from the specific cancer investigated.

Consequently, the antibodies of the invention are most useful in assays of the serum from subjects aimed at detecting the presence of cancer.

As the antibodies of the invention detect antigens found predominantly in human mammary carcinomas, they are useful in determining the cellular lineage from which human mammary carcinomas arise and has potential clinical utility in breast cancer diagnosis and therapy, for example in the preparation of medicaments.

## DETAILED DESCRIPTION OF THE INVENTION

Monoclonal antibodies to the breast carcinoma cell line MCF-7 was produced using the technique of Kohler and Milstein supra. The mammary cell line MCF-7 (Soule H.D. et al. JNCI, 51 (1973) 1409-1413) was cultured in RPMI-1640 supplemented with 2 mM L-glutamine, 1% non-essential amino acids and 10% fetal calf serum.

RBF-mice (female, aged 8 weeks) were immunized with either 100 µl 100x concentrated serum-free conditioned medium or 100 µg MCF-7 cells from the subline no. 9 of the MCF-7 cell line (Briand, P., and Lykkesfeldt, A.E. Long term cultivation of human breast cancer cell line, MCF-7, in a chemically defined medium. Effect of estradiol. Anticancer Res., 6:85-90, 1986), which has been adapted to growth in a chemically defined medium, solubilized in 15 mM deoxycholate injected subcutaneously. The first injection was given in complete Freund's adjuvant, a second and a third injection were given in Freund's incomplete adjuvant with two weeks intervals, and two weeks after the third injection the animals were boosted with either MCF-7 serum-free supernatant or detergent solubilized T-47D cells (Keydar, I., Chen, L., Karby, S., Weiss, F.R., Delarea, J., Radu, M. Chaitcik, S., and Brenner, H.J. Establishment and characterization of a cell line of human breast carcionoma origin. Eur.J.Cancer, 15:659-670, 1979) injected intravenously. Three days after the boost the animals were sacrificed and their spleens were taken. A spleen cell suspension was prepared, and the resulting cell suspension was washed by two centrifugations (800 x g) in protein-free RPMI 1640).

Because the antibody-producing cells obtained from the spleen do not independently reproduce, and thus cannot be cultured, they are fused with cells which may be independently cultured either in vivo or in vitro so that the genetic and metabolic processes of the fused hybridomas have characteristics of each of the parent cells, and it is intended that certain of the cells obtained will have the capability to independently reproduce and to produce the antibody of the antibody-producing parent cell. Some tumor cells, particularly myeloma cells, may be advantageously fused with antibody-producing cells to produce hybridomas. Although it is not necessary, it is preferred that the tumor cells and antibody-producing cells be derived from the same species to enhance the likelihood that the genetic and biochemical properties of the parent cells will be compatible and thus produce viable and stable hybridomas. A number of myeloma cultures have been characterized, and herein, mouse-derived, non-antibody-producing myeloma cell line, FOX-NY that was obtained from HyClone Laboratories, Utah, USA (R.T. Taggart and I.M. Samloff: Science 219 (1983) 1228-1230) was used to produce the hybridomas. It is to be understood that other tumor lines also may be used.

It is advantageous to select a myeloma line which does not produce antibody so that the resulting hybridoma will only produce the antibody of the parent spleen of lymph node cell. This is particularly important when the antibody is used for therapeutic purposes, e.g., as a cytotoxic agent against tumor cells, where it is undesirable to introduce extraneous antibodies which could produce side reactions.

Prior to the fusion the FOX-NY myeloma cell line was grown in RPMI-1640 supplemented with 2 mM L-glutamine, 1% non-essential amino acids and 10% fetal calf serum.

Spleen cells from 1 of the immunized mice (1x10$^8$) cells) were fused with cells of the FOX-NY myeloma cell line which is deficient in the selectable enzyme marker loci adenosine phosphoribosyl transferase (APRT-) and hypoxanthine phosphoribosyl transferase (HPRT-). Thus, the exposure of cell fusion mixtures to a medium requiring APRT-activity (APRT+ selection) eliminates both unfused APRT- myelomas and APRT- hybridomas (R.T. Taggart and I.M. Samloff supra). The fused cells were seeded on Balb/C-strain mouse macrophage feeder layers in a total of ten 96-well microtiter plates (NUNC, Roskilde, Denmark) in the above medium further supplemented with 7.5 x 10-5 M adenine, 8 x 10-7 M aminopterin, and 1.6 x 10-5 M thymidin (AAT selection medium). In total 12 fusions were performed.

Clones of hybridomas may be grown in vitro according to known tissue culture techniques such as is described by Cotten et al., Eur.J.Immunol. 3 136 (1973). Alternatively, hybridomas may be grown in vivo as tumors in a histocompatible animal or in athymic nude mice. The antibodies may be recovered from the in vitro culture medium (the supernatant of the clone) or from the serum or ascitic fluid of the animal by means known in the art, e.g., Gehard et al., Proc.Natl.Acad.Sci., 75, pp. 1510-1514 (1978). In some cases it may be advantageous to obtain the antibodies directly from the cells of the culture or tumor.

The initial specificity screening of hybridoma supernatants using glutaraldehyde fixed MCF-7, T47/D, HMT 3522, HBL 100, HH 456, and Daudi (MCF-7 and T47/D are mammary carcinoma cell lines, HMT 3522 and HBL 100 are nontumorigenic mammary cell lines, Hu 456 is a normal urothelium cell, and Daudi is a human Burkitt Lymphoma cell line which all were cultured in the manner that MCF-7 was cultured) as target cells was performed by ELISA assay. Hybrids which reacted with either MCF-7 and/or T47/D (but not the other cells) were chosen for a second screening.

Selected hybridomas were cultured in RPMI-1640 supplemented with 2 mM L-glutamine, 1% non-essential amino acids, 10% fetal calf serum, and the AAT selection medium.

When a useful hybridoma clone is produced it is generally advantageous to reclone the cell line to avoid overgrowth of cultures with variant cells no longer producing antibody. Because the hybridoma contains some, but not all, of the genetic material of each parent cell, the full characteristics of the hybridoma are not known. Often a hybridoma clone, due to original genetic deficiency or subsequent chromosome loss, after several passages may lose its ability to reproduce and/or to produce the particular antibody. Accordingly, it is important, soon after the initial hybridization, that a hybridoma clone of interest is recloned to insure the availability of functioning strains of the antibody-producing hybridoma. By recloning is meant fusing a hybridoma cell with neoplastic cells, e.g. to isolate individual hybridoma cells and expand them into cultures which are clones.

Three cell line cultures initially designated as E1, S3, and S8 and their reclones (especially two reclones of S8 designated S8IgM and S8IgG, which produce two different immunoglobulin isotypes, IgM and IgG respectively with identical specificity) produce monoclonal antibodies of which E1 is specific for a cytoplasmatic antigen, and S3 and S8IgM and S8IgG are specific for two cell surface antigens that occur predominantly on human mammary carcinoma cells.

Stable subclones of the cell lines E1, S3, S8IgM, and S8IgG are on deposit at the European Collection of Animal Cell Cultures (ECACC) PHLS Centre for Applied Microbiology and Research, Porton Down, Salisbury, Wiltshire SP4 OJG, UK, for the purposes of patent procedure on the date indicated below. ECACC being an international depositary authorized under the Budapest Treaty, affords permanence of the deposits in accordance with rule 9 of the Treaty.

| Deposit date | Depositors reference | ECACC designation |
|---|---|---|
| 10 Nov. 1986 | Hybridoma cell line E1 | 86 11 10 05 |
| 10 Nov. 1986 | Hybridoma cell line S8IgM | 86 11 10 04 |
| 10 Nov. 1986 | Hybridoma cell line S8IgG | 86 11 10 03 |
| 10 Nov. 1986 | Hybridoma cell line S3 | 86 11 10 02 |

The isotype of monoclonal antibody was determined by Ouchterlony gel immunodiffusion with rabbit antiserum to mouse IgG1, IgG2a, IgG2b, IgG3, IgM and IgA (Serotek, UK). The monoclonal antibodies of the inventions were determined to be of the IgG1 and M isotypes, as indicated in table I below. The antibodies are characterized in that S3, S8IgM and S8IgG react with surface antigens present in nearly all breast cancers examined and E1 with a cytoplasmatic antigen, but essentially not with normal breast epithelium. They react with some other normal tissues and with a number of adenocarcinomas not of breast origin. They are therefore suitable for the classification diagnosis of these cancers from metastatic breast cancer. It is anticipated that they will slow down the growth of cells of the MCF-7 human breast cancer line in vitro.

REACTIVITY

The reactivity of the antibodies of the invention was tested according to the following procedure:

Wells in immunoplates (NUNC, Denmark) were pretreated with 1 µg poly-L-lysine per ml in PBS for 30 min. Cells were washed 3 times in PBS and 50 µl/well plated at a concentration of $1 \times 10^6$ cells per ml. After centrifugation the cells were fixed with 0.025% glutaraldehyde in PBS for 10 min. After 3 successive washes with PBS-T, cells were incubated with PBS containing 2% BSA and 0.1% glycine for 1 h. Wells were incubated with hybridoma supernatants (50 µl) for 1 h. Plates were washed 3 times with PBS-T, followed by incubation with peroxidase conjugated goat anti-mouse antibodies (TAGO, U.S.A.) diluted 1:3000 in PBS plus 2% BSA. Plates were washed five times and incubated 10 min with enzyme substrate (0.4 mg/ml O-phenylenediamine; 0.05% $H_2O_2$ in 50 mM citric acid buffer). The enzyme reaction was terminated by addition of 150 µl 2N $H_2SO_4$. The absorbance was determined by a Kontron SLT 210 ELISA reader (SLT Lab. Instruments, Austria). Endogenic peroxidase of erythrocytes and bone marrow cells was blocked by incubation with methanol; 0.3% $H_2O_2$ for 30 min.

The antibodies of the invention were tested against the following cell lines:

The cell lines Hu456 and Hu609 from human urothelium (Christensen, B., Kieler, KJ., Vilien, M., Don, P., Wang, C.Y., and Wolf, H. A classification of human urothelial cells propagated in vitro. Anticancer Res., 4:319-338, 1984); the ovariam teratocarcinoma cell line PA-1 (Zeuthen, J., Nørgaard, J.O.R., Avner, P., Fellous, M., Wartiovaara, J., Vaheri, A., Rosen, A., and Giovanella, B.C. Characterization of a human ovarian teratocaricinoma-derived cell line. Int.J.Cancer, 25:19-32, 1980), the testicular teratocarcinoma cell line NT-2, the Burkitt lymphoma cell line DAUDI (Klein, E., and Klein, G., Cancer Res., 28, 1300-1310, 1968.), the melanoma cell line Bowes and 3T3 mouse embryo fibroblasts and the RH-SLC11 squamous cell lung carcinoma cell line (Olsson, L., Sorensen, H.S., and Behnke, O. Intratumoral phenotypic diversity of cloned human lung tumor cell lines and consequences for analyses with monoclonal antibodies. Cancer, 54:1757-1764, 1984) and bone marrow cells,A, B and O erythrocytes and lymphocytes obtained from volunteers.

The results of the reactivity evaluation and isotype determination are shown in table I below:

TABLE I

Reactivity of monoclonal antibodies in cell-binding ELISA

| | E1 | S3 | S8 | |
| | | | S8IgG | S8IgM |
|---|---|---|---|---|
| Isotype | M | M | $G_1$ | M |
| IF[X] | cytoplas-matic | membrane | | membrane |
| Cell lines | | | | |
| MCF-7 | ·3 | 3 | 2 | 2 |
| T47/0 | 3 | 2 | 0 | 0 |
| HBL-100 | 0 | 0 | 0 | 0 |
| 3522 | 0 | 0 · | 0 | 0 |
| RH-SLC44 | 3 | 1 | 0 | 0 |
| NT-2 | 1 | 0 | 0 | 0 |
| PA-1 | 0 | 0 | 0 | 0 |
| Bowes | 0 | 0 | 0 | 0 |
| Hu456 | 0 | 0 | 0 | 0 |
| Hu609 | 0 | 0 | 0 | 0 |
| 3T3 | 0 | 0 | 0 | 0 |
| Daudi | 0 | 0 | 0 | 0 |
| Lymphocytes | 0 | 0 | 0. | 0 |
| Bone marrow cells | 0 | 0 | 0 | 0 |
| A, B, O erythrocytes | 0 | 0 | 0 | 0 |

O: $A_{492} < 0.150$;  1: $0.150 < A_{492} < 0.500$;  2: $0.500 < A_{492} < 1.000$;  3: $1.000 < A_{492}$

IF = immunofluorescence

MAbs generated with membrane extract are designated E, MAbs generated with MCF-7 serum-free supernatant are designated S.

## Claims

1. A cell line produced by the fusion of an antibody-producing animal cell and a neoplastic cell, which cell line produces an antibody specific for a cell surface antigen found prodominanatly in human mammary carcinoma cells.

2. A cell line as claimed in Claim 1 wherein the antibody-producing cell is derived from a rodent.

3. A cell line as claimed in Claim 2 wherein the antibody-producing cells are rodent spleen cells or rodent lymph node cells.

4. A cell line as claimed in Claim 3 wherein the antibody-producing cells are mouse spleen cells.

5. A cell line as claimed in any one of the preceding claims wherein the neoplastic cell is a non-antibody producing cell.

6. A cell line as claimed in Claim 1 which is one of the hybridomas S3, S8 IgM, S8 IgG or E1 or an antibody-producing reclone thereof.

7. A monoclonal antibody specific for a cell surface antigen found predominantly in human mammary carcinoma cells which is produced by the cell line of any of Claims 1 to 6.

8. A monoclonal antibody as claimed in Claim 7 which is obtained from one of the hybridoma cultures S3, S8 IgM, S8 IgG or E1 or antibody-producing reclones thereof.

9. A monoclonal antibody according to Claim 7 or 8 in suitably labelled form for use in radioimmunoimaging.

10. Use of a monoclonal antibody according to Claim 7, 8 or 9 as a therapeutic agent.

11. A method of producing monoclonal antibodies specific for a cell surface antigen found predominantly in human mammary carcinoma cells, which method comprises inoculating an animal with material containing the antigen, obtaining antibody-producing cells from the animal, fusing the antibody-producing cells and neoplastic cells to produce hybridomas. Selecting those hybridomas that produce an antibody which reacts with most human mammary tumor tissues and is generally unreactive with other human tumor tissues, producing clones of the selected hybridomas, and collecting the antibody produced thereby.

12. A method as claimed in Claim 11 wherein the antibody-producing cells are RBF mouse spleen cells.

13. A method as claimed in Claim 11 or 12 wherein the antigen-containing material is derived from the human mammary carcinoma cell line MFC-7.

14. A method as claimed in any one of the preceding Claims 11 to 14 wherein the neoplastic tissue cells are of the myeloma cell line FOX-NY.

15. A method as claimed in any one of the preceding Claims 11 to 13 wherein the clones are grown in host animals, and the antibody is obtained from the host animal's blood serum or the host animal's ascitic fluid.

16. A method as claimed in any one of Claims 11 to 14 wherein the clones are grown in vitro, and the antibody is obtained from culture supernatants of the clones.

17. An antigen containing an epitope which is recognized by the antibody produced by one of the hybridoma cell lines S3, S8 IgG, S8 IgM or E1 or immunologically equivalent monoclonal antibodies.

18. The antigen of Claim 17 wherein the epitope is an anti-idiotype antibody moiety or an immunological equivalent thereof.

19. A method for the detection of human mammary carcinoma which comprises contacting a source containing or possibly containing an antigen specific for human mammary carcinoma with the monoclonal antibody of Claim 7, 8 or 9 and detecting whether said antibody binds to said antigen.

20. A diagnostic kit for the detection in serum, in tissue or tissue extracts of human mammary carcinoma comprising the monoclonal antibody of Claim 7, 8 or 9.

21. The diagnostic kit of Claim 20, adapted for dot-blot analysis, an immunometric sandwich assay or a competitive assay.

22. The diagnostic kit of Claim 21 adapted for immunohistochemical localization of human mammary carcinoma cells.

23. A diagnostic kit comprising a carrier being compartmentalized to receive in close confinement therein two or more containers,

a first container comprising an antigen containing an epitope recognized by the antibody produced by one of the hybridomas S3, S8 JgG, S8 JgM or E1, and

a second container comprising a detectably labelled, monoclonal antibody which binds an epitope specific for human mammary carcinoma which epitope is produced by the cell line MCF-7.

24. The kit of Claim 23, wherein said antibody in said second container is produced by one of the hybridoma cell lines S3, S8 IgG, S8 IgM or E1.